# EUROPEAN PATENT SPECIFICATION

(11) **EP 3 082 727 B1**
(45) Date of publication and mention of the grant of the patent: **22.07.2020**
(21) Application number: 14814871.1
(22) Date of filing: 18.12.2014
(51) Int. Cl.: A61K 8/31, A61K 8/34, A61K 8/46, A61K 8/55, A61Q 5/04, A61Q 9/04, A61K 8/86

(54) **NON-COLORING COMPOSITION FOR RESHAPING OR REMOVING HAIR**
NICHT FÄRBENDE ZUSAMMENSETZUNG ZUR HAARUMFORMUNG ODER HAARENTFERNUNG
COMPOSITION NON COLORANTE POUR LA DÉFORMATION OU LA DÉPILATION DES CHEVEUX

(30) Priority: 19.12.2013 FR 1362961
(43) Date of publication of application: 26.10.2016
(73) Proprietor: L'OREAL, 75008 Paris (FR)
(72) Inventor: MARIO, Maud, F-75017 Paris (FR); ROULET, Charlotte, F-75020 Paris (FR); SELLIER, Céline, F-75011 Paris (FR); SIMONET, Frédéric, 93400 SAINT-OUEN (FR); KRAVTCHENKO, Sylvain, F-16200 Sigogne (FR)
(74) Representative: L'Oreal
(86) International application number: PCT/EP2014/078386
(87) International publication number: WO 2015/091743

(56) References cited:
- EP-A1- 0 302 265
- WO-A1-2011/024300
- JP-A- 2006 076 922
- JP-A- 2008 290 971
- Anonymous: "SAFETY DATA SHEET. CRODAFOS CES-PA-(RB)", CRODA , 29 November 2013 (2013-11-29), XP002728953, Retrieved from the Internet: URL:http://msds.crodadirect.com/MSDSdownlo ad.aspx?docID=b00f5dbc-127c-4f61-a9b2-f972 d47d58a1 [retrieved on 2014-08-26]
- "International Cosmetic Ingredient Dictionary and Handbook", 2010, Personal Care Products Council, XP002728954, vol. 2, pages 1825-1826, entry "MYRISTYL ALCOHOL", paragraph "Reported Functions"

## Description

The present invention relates to a non-colouring composition comprising one or more sulfureous, preferably thiol-based, reducing agents, one or more phosphoric surfactants, one or more nonionic surfactants other than the phosphate surfactants, and one or more non-liquid fatty substances in a particular content.

The invention also relates to a process for permanently reshaping keratin fibres, in particular human keratin fibres such as the hair, comprising the use on the said fibres of the composition defined above and optionally of an oxidizing composition.

Many people are dissatisfied with the appearance of their hair; in particular, people who have curly hair usually wish to obtain straight hair, and, conversely, people who have curl-free hair wish to have curly hair.

The techniques used for permanently reshaping the hair generally consist in applying a reducing composition containing a reducing agent, generally a thiol-based reducing agent, at neutral or moderately alkaline pH values, below 10.

Similarly, hair-removing compositions may comprise a reducing agent, often a thiol-based reducing agent, at very high pH values, generally above 11.

Reducing agents generally have a strong odour, which is found on application to various keratin substrates. This is particularly experienced during application to the hair and while they are left to stand on the hair.

These reducing agents may also lead to degradation of the hair during permanent reshaping treatments.

There is thus a real need to use compositions, which are intended to be used during a process for permanently reshaping keratin fibres or in a hair-removing composition, which do not have all the drawbacks described above, i.e. which give off less odour so as to make the applications of the compositions more comfortable for users.

It has already been proposed to reduce the unpleasant odour of permanent reshaping compositions. Documents JP2006076922 and JP2008290971 describe, for example, compositions for permanently reshaping the hair, which comprise a phosphoric surfactant. The performance qualities in terms of odour reduction are, nevertheless, insufficient.

There is thus a need to use compositions, which are intended to be used during a process for permanently reshaping keratin fibres or in a hair-removing composition, which generate even less unpleasant odour.

The Applicant has discovered, surprisingly, that it is possible to achieve the desired properties by combining, in a non-colouring composition, one or more sulfureous reducing agents, one or more phosphoric surfactants, one or more nonionic surfactants other than the phosphoric surfactants, and at least 5.0% of non-liquid fatty substance(s).

The composition according to the invention makes it possible to reduce the evolution of ammonia and of volatile sulfureous compounds during the application to keratin substrates, which limits the odours perceived.

Moreover, in the context of compositions intended to be used during a process for permanently reshaping keratin fibres, good friendliness towards the integrity of the fibres is found.

A first subject of the present invention is thus a non-colouring composition comprising:
- one or more sulfureous reducing agents, the sulfureous reducing agent(s) being present in proportions ranging from 1.0% to 50.0% by weight relative to the total weight of the composition;
- one or more phosphoric surfactants;
- one or more nonionic surfactants other than the phosphoric surfactants; and
- from 7% to 60% by weight of non-liquid fatty substance(s), relative to the total weight of the composition, the nonionic surfactant(s) being different from said non-liquid fatty substance(s), wherein the non-liquid fatty substance(s) comprises fatty alcohols;
- one or more colouring agents which content, if present, does not exceed 0.001 % by weight, relative to the total weight of the composition.

A subject of the invention is also a process for permanently reshaping, especially for straightening or perming, keratin fibres, in particular human keratin fibres such as the hair, which consists in performing the following steps:
(i) a composition as defined previously is applied to the said fibres and is left to stand on the fibres for a time sufficient for shaping, and
(ii) an oxidizing composition is optionally applied to the said fibres for a time sufficient for fixing the shape.

Finally, a subject of the invention is also the use of the composition defined previously for permanently reshaping the hair or for hair removal.

The composition according to the invention is non-colouring, i.e. it does not give the keratin fibres a visible colouring after removal of the said composition. Preferably, it does not comprise any colouring agent.

According to the present invention, the term "colouring agents" means agents for colouring keratin fibres such as direct dyes or oxidation dye precursors (bases and couplers). If they are present, their content does not exceed 0.001% by weight, relative to the total weight of the composition. Specifically, at such a content, only the composition would be dyed, i.e. no dyeing effect would be observed on the keratin fibres.

It is recalled that oxidation dye precursors, oxidation bases and couplers are colourless or sparingly coloured compounds, which, via a condensation reaction in the presence of an oxidizing agent, give a coloured species. With regard to direct dyes, these compounds are coloured and have affinity for keratin fibres.

As indicated above, the composition according to the invention comprises one or more sulfureous reducing agents, preferably chosen from the reducing agents of formula:

H(X')_{q}(R')ᵣ

in which X' represents S or SO₂, q is 1, r is 1 or 2 or 3, and R' is a linear, branched, saturated or unsaturated C₁-C₂₀ hydrocarbon-based radical, optionally interrupted with a heteroatom, and optionally comprising substituents chosen from a hydroxyl group, a halogenated group, an amine group, a salified or non-salified carboxyl group, a (C₁-C₃₀ alkoxy)carbonyl group, an amido group, a (C₁-C₃₀ alkyl)aminocarbonyl group, a (C₁-C₃₀ acyl)amino group, a monoalkylamino or dialkylamino group, or a monohydroxyamino or dihydroxyamino group, or a salt thereof in combination with a base.

The sulfureous reducing agent(s) used in the composition according to the invention are chosen from thiol-based and non-thiol-based reducing agents.

As thiol-based reducing agents that may be used in the composition according to the invention, mention may be made of thiol-based reducing agents chosen from thioglycolic acid, thiolactic acid, cysteine, cysteamine, homocysteine, glutathione, thioglycerol, thiomalic acid, 2-mercaptopropionic acid, 3-mercaptopropionic acid, thiodiglycol, 2-mercaptoethanol, dithiothreitol, thioxanthine, thiosalicylic acid, thiopropionic acid, lipoic acid and N-acetylcysteine, and salts thereof.

As non-thiol-based reducing agents that may be used in the composition according to the invention, mention may be made especially of alkali metal or alkaline-earth metal sulfites.

Preferably, the sulfureous reducing agent(s) used in the composition according to the invention are thiol-based reducing agents, in particular thioglycolic acid and thiolactic acid or salts thereof, especially alkali metal, alkaline-earth metal or ammonium salts thereof, or cysteine, and mixtures thereof.

Preferentially, the sulfureous reducing agent used in the composition according to the invention is thioglycolic acid and salts thereof, especially ammonium thioglycolate or potassium thioglycolate, or cysteine.

The sulfureous reducing agent(s) may be present in the composition according to the invention in a content ranging from 1% to 50% by weight, or 1.0% to 20.0% by weight, preferably in a content ranging from 2.0% to 15.0% by weight relative to the total weight of the composition.

The composition according to the invention comprises at least one phosphoric surfactant.

The term "phosphoric surfactant" means a surfactant whose polar part comprises at least one phosphorus atom.

The phosphoric surfactant may have the following formula: 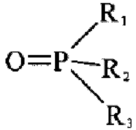 in which:
R₁, R₂ and R₃, which may be identical or different, represent a group chosen from:
- a group -OM, in which M represents a hydrogen atom or an alkali metal, such as Na, Li or K, preferably Na or K;
- a group -OR₄, in which R₄ represents a linear or branched C₁-C₄₀ alkyl group, preferably a C₁₂-C₂₀ alkyl group and more preferably a C₁₆ or C₁₈ alkyl group, a linear or branched C₂-C₄₀ alkenyl group, preferably a C₁₂-C₂₀ alkenyl group and more preferably a C₁₆ or C₁₈ alkenyl group, a C₃-C₄₀ cyclic alkyl group, a C₃-C₄₀ cyclic alkenyl group, a C₅-C₄₀ aromatic group or a C₆-C₄₀ aralkyl group; and
- an oxyalkylene group -(OCH₂CH₂)ₙ(OCH₂CH(CH₃))ₘOR₄ in which R₄ is as defined previously, n represents an integer ranging from 1 to 50 and m represents an integer ranging from 0 to 50,
given that at least one from among R₁, R₂ and R₃ is a group -OM and that at least one from among R₁, R₂ and R₃ is a group -OR₄ or -(OCH₂CH₂)ₙ(OCH₂CH(CH₃))ₘOR₄.

Preferably, the phosphoric surfactant may be chosen from alkoxylated fatty alcohol phosphates containing from 12 to 20 carbon atoms with from 1 to 50 mol of alkylene oxide chosen from ethylene oxide and propylene oxide, and non-alkoxylated alcohol dialkyl phosphates containing from 12 to 22 carbon atoms, and mixtures thereof. The alkyl group of the fatty alcohol or of the non-alkoxylated alcohol may be linear or branched, or a saturated or unsaturated alkyl group.

Preferably, use may be made of a combination of at least one oxyalkylenated phosphoric surfactant and of at least one non-oxyalkylenated phosphoric surfactant.

More preferably, the combination of phosphoric surfactants may be chosen from the group consisting of a combination of ceteth-10 phosphate and dicetyl phosphate, a combination of ceteth-20 phosphate and dicetyl phosphate, and a combination of oleth-5 phosphate and dioleyl phosphate.

As product comprising the combination of ceteth-10 phosphate and dicetyl phosphate, mention may be made of Crodafos CES or Crodafos CES-PA, sold by Croda. As product comprising the combination of ceteth-20 phosphate and dicetyl phosphate, mention may be made of Crodafos CS-20 Acid, sold by Croda. As product comprising the combination of oleth-5 phosphate and dioleyl phosphate, mention may be made of Crodafos HCE, sold by Croda.

The phosphoric surfactant(s) may be present in the composition according to the invention in a content ranging from 0.01% to 20.0% by weight, preferably in a content ranging from 0.1% to 15.0% by weight and better still in a content ranging from 0.2% to 10.0% by weight relative to the total weight of the composition.

Preferably, the ratio by weight between the amount of sulfureous reducing agent(s), on the one hand, and the amount of phosphoric surfactant(s), on the other hand, is greater than or equal to 0.5 and preferably ranges from 1 to 30 and more preferably from 1 to 20.

The composition according to the invention also comprises a nonionic surfactant other than phosphoric surfactants.

Examples of nonionic surfactants that may be used in the composition according to the invention are described, for example, in the Handbook of Surfactants by M.R. Porter, published by Blackie & Son (Glasgow and London), 1991, pp. 116-178.

As examples of nonionic surfactants other than phosphoric surfactants, mention may be made of the following nonionic surfactants, alone or as mixtures:
- oxyalkylenated (C₈-C₂₄)alkylphenols;
- saturated or unsaturated, linear or branched, oxyalkylenated or glycerolated C₈-C₃₀ alcohols;
- saturated or unsaturated, linear or branched, oxyalkylenated C₈-C₃₀ fatty acid amides;
- esters of saturated or unsaturated, linear or branched, C₈-C₃₀ acids and of polyethylene glycols;
- preferably oxyethylenated esters of saturated or unsaturated, linear or branched, C₈-C₃₀ acids and of sorbitol;
- fatty acid esters of sucrose;
- (C₈-C₃₀)alkylpolyglycosides, (C₈-C₃₀)alkenylpolyglycosides, which are optionally oxyalkylenated (0 to 10 oxyalkylene units) and which comprise 1 to 15 glucose units, (C₈-C₃₀)alkylglucoside esters;
- saturated or unsaturated oxyethylenated plant oils;
- condensates of ethylene oxide and/or of propylene oxide, inter alia, alone or as mixtures;
- N-(C₈-C₃₀)alkylglucamine derivatives and N-(C₈-C₃₀)acyl-methylglucamine derivatives;
- aldobionamides;
- amine oxides;
- oxyethylenated and/or oxypropylenated silicones.

The oxyalkylene units are more particularly oxyethylene or oxypropylene units, or a combination thereof.

The number of moles of ethylene oxide and/or of propylene oxide preferably ranges from 1 to 100 and more particularly from 2 to 50; the number of moles of glycerol ranges in particular from 1 to 30.

By way of example of glycerolated nonionic surfactants, use may preferably be made of monoglycerolated or polyglycerolated C₈-C₄₀ alcohols comprising from 1 to 30 mol of glycerol, preferably from 1 to 10 mol of glycerol.

As examples of compounds of this type, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

Among the glycerolated alcohols, it is more particularly preferred to use the C₈/C₁₀ alcohol containing 1 mol of glycerol, the C₁₀/C₁₂ alcohol containing 1 mol of glycerol and the C₁₂ alcohol containing 1.5 mol of glycerol.

Preferably, the nonionic surfactant(s) are chosen from:
- oxyethylenated and/or oxypropylenated C₈-C₃₀ alcohols comprising from 1 to 100 mol of ethylene oxide and/or of propylene oxide, preferably from 2 to 50 and more particularly from 2 to 30 mol of ethylene oxide and/or of propylene oxide;
- saturated or unsaturated oxyethylenated plant oils comprising from 1 to 100 and preferably from 2 to 50 mol of ethylene oxide;
- (C₈-C₃₀)alkyl(poly)glycosides, which are optionally oxyalkylenated (0 to 10 OE) and comprising 1 to 15 glucose units;
- monoglycerolated or polyglycerolated C₈-C₄₀ alcohols, comprising from 1 to 30 mol of glycerol and preferably from 1 to 10 mol of glycerol;
- saturated or unsaturated, linear or branched, oxyalkylenated C₈-C₃₀ fatty acid amides;
- mixtures thereof.

Even more preferentially, the nonionic surfactants other than the phosphoric surfactants are chosen from oxyethylenated and/or oxypropylenated C₈-C₃₀ alcohols comprising from 1 to 100 mol of ethylene oxide and/or of propylene oxide, preferably from 2 to 50 and more particularly from 2 to 30 mol of ethylene oxide and/or of propylene oxide.

Advantageously, the content of nonionic surfactant(s) ranges from 0.01% to 30.0% by weight, preferably from 0.1% to 20.0% by weight and more preferably from 1.0% to 10.0% by weight relative to the total weight of the composition.

The composition according to the invention also comprises one or more fatty substances comprising fatty alcohols, that are not liquid at room temperature (25°C) and at atmospheric pressure (760 mmHg, i.e. 1.013 × 10⁵ Pa).

The term "fatty substance" means an organic compound that is insoluble in water at ordinary room temperature (25°C) and at atmospheric pressure (760 mmHg, i.e. 1.013 × 10⁵ Pa), with a solubility in water of less than 5%, preferably less than 1% and even more preferentially less than 0.1%.

In addition, the fatty substances are generally soluble in organic solvents under the same temperature and pressure conditions, for instance chloroform, ethanol, benzene, liquid petroleum jelly or decamethylcyclopentasiloxane.

The term "non-liquid fatty substance" means a solid compound or a compound that has a viscosity of greater than 2 Pa.s at a temperature of 25°C and at a shear rate of 1 s⁻¹.

In a first variant of the invention, the non-liquid fatty substances are chosen from non-silicone non-liquid fatty substances. Preferably, the non-silicone non-liquid fatty substances are chosen from fatty alcohols, fatty acid and/or fatty alcohol esters, non-silicone waxes, fatty amines and fatty ethers, which are non-liquid and preferably solid.

More particularly, the non-liquid fatty alcohols according to the invention are chosen from linear or branched, saturated or unsaturated alcohols containing from 8 to 30 carbon atoms.

Examples that may preferably be mentioned include cetyl alcohol, stearyl alcohol and a mixture thereof (cetylstearyl alcohol). Cetylstearyl alcohol is more particularly used.

The non-liquid esters of fatty acids and/or of fatty alcohols that may be used in the composition according to the invention are generally chosen from solid esters derived from C₉-C₂₆ fatty acids and from C₉-C₂₆ fatty alcohols.

Examples that may preferably be mentioned include octyldodecyl behenate, isocetyl behenate, cetyl lactate, stearyl octanoate, octyl octanoate, cetyl octanoate, decyl oleate, myristyl stearate, octyl palmitate, octyl pelargonate, octyl stearate, alkyl myristates such as cetyl myristate, myristyl myristate and stearyl myristate, and hexyl stearate.

The non-silicone wax(es) are chosen especially from carnauba wax, candelilla wax, esparto wax, paraffin wax, ozokerite, plant waxes, such as olive tree wax, rice wax, hydrogenated jojoba wax or absolute flower waxes, such as the blackcurrant blossom essential wax sold by Bertin (France), or animal waxes, such as beeswaxes or modified beeswaxes (cerabellina), and ceramides.

The solid amides that may be used in the composition according to the invention are chosen from ceramides or ceramide analogues, such as the natural or synthetic glycoceramides corresponding to formula (III) below: 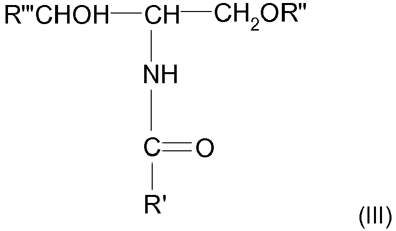 in which:
- R' denotes a linear or branched, saturated or unsaturated alkyl radical, derived from C₁₄-C₃₀ fatty acids, it being possible for this radical to be substituted with a hydroxyl group in the alpha-position, or a hydroxyl group in the omega-position esterified with a saturated or unsaturated C₁₆-C₃₀ fatty acid;
- R" denotes a hydrogen atom or a radical (glycosyl)n, (galactosyl)m or sulfogalactosyl, in which n is an integer ranging from 1 to 4 and m is an integer ranging from 1 to 8;
- R''' denotes a C₁₅-C₂₆ hydrocarbon-based radical, saturated or unsaturated in the alpha-position, it being possible for this radical to be substituted with one or more C₁-C₁₄ alkyl radicals;
with the proviso that, in the case of natural ceramides or glycoceramides, R''' may also denote a C₁₅-C₂₆ alpha-hydroxyalkyl radical, the hydroxyl group being optionally esterified with a C₁₆-C₃₀ alpha-hydroxy acid.

The ceramides that are preferred in the context of the present invention are those described by Downing in Arch. Dermatol., Vol. 123, 1381-1384, 1987, or those described in French patent FR 2 673 179.

The ceramide(s) more particularly preferred that may be used in the composition according to the invention are the compounds for which R' denotes a saturated or unsaturated alkyl derived from C₁₆-C₂₂ fatty acids; R" denotes a hydrogen atom; and R''' denotes a linear, saturated C₁₅ radical.

Preferentially, the following compounds may especially be chosen: N-linoleoyldihydrosphingosine, N-oleoyldihydrosphingosine, N-palmitoyldihydrosphingosine, N-stearoyldihydrosphingosine, N-behenoyldihydrosphingosine, and a mixture of these compounds.

Even more preferentially, ceramides are used for which R' denotes a saturated or unsaturated alkyl radical derived from fatty acids, R" denotes a galactosyl or sulfogalactosyl radical and R''' denotes a -CH=CH-(CH₂)₁₂-CH₃ group.
Other waxes or waxy starting materials that may be used according to the invention are especially marine waxes such as those sold by the company Sophim under the reference M82, and waxes of polyethylene or of polyolefins in general.

The non-liquid fatty ethers that may be used in the composition according to the invention are chosen from dialkyl ethers and especially dicetyl ether and distearyl ether, alone or as a mixture.

In a second variant of the invention, the non-liquid fatty substance(s) may be chosen from silicone non-liquid fatty substances, such as silicone gums or resins.

The silicone gums that may be used in accordance with the invention are especially polydialkylsiloxanes and preferably polydimethylsiloxanes with high number-average molecular weights ranging from 200 000 to 1 000 000, used alone or as a mixture in a solvent.

The said solvent may be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Products that may be used more particularly in accordance with the invention are mixtures such as:
- the mixtures formed from a hydroxy-terminated polydimethylsiloxane or dimethiconol (CTFA) chain, and from a cyclic polydimethylsiloxane, also known as cyclomethicone (CTFA), such as the product Q2 1401 sold by the company Dow Corning;
- mixtures of a polydimethylsiloxane gum and a cyclic silicone, such as the product SF 1214 Silicone Fluid from the company General Electric; this product is an SF 30 gum corresponding to a dimethicone, having a number-average molecular weight of 500 000, dissolved in the oil SF 1202 Silicone Fluid corresponding to decamethylcyclopentasiloxane;
- mixtures of two PDMSs with different viscosities, and more particularly of a PDMS gum and of a PDMS oil, such as the product SF 1236 from the company General Electric. The product SF 1236 is a mixture of a gum SE 30 defined above, with a viscosity of 20 m²/s and of an oil SF 96 with a viscosity of 5×10⁻⁶ m²/s. This product preferably comprises 15% of gum SE 30 and 85% of an oil SF 96.

The organopolysiloxane resins that may be used in accordance with the invention are crosslinked siloxane systems containing the following units:

(R₇)₂SiO_{2/2}, (R₇)₃SiO_{1/2}, R₇SiO_{3/2} and SiO_{4/2}

in which R₇ represents an alkyl containing 1 to 16 carbon atoms. Among these products, the ones that are particularly preferred are those in which R₇ denotes a C₁-C₄ lower alkyl group, more particularly methyl.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethylsiloxysilicate type resins sold in particular under the names X22-4914, X21-5034 and X21-5037 by the company Shin-Etsu.

Preferably, the non-liquid fatty substance(s) are non-silicone and even more preferentially are chosen from fatty alcohols, even more preferentially from cetyl alcohol, stearyl alcohol and mixtures thereof such as cetylstearyl alcohol.

The composition according to the invention comprises one or more non-liquid fatty substance(s) comprising fatty alcohols, in a content ranging from 7.0% to 60.0% by weight, better still from 7.0% to 30.0% by weight, preferably from 7.0% to 25.0% by weight and more preferably from 8% to 25% by weight relative to the total weight of the composition.

The composition according to the invention may also comprise one or more fatty substances that are liquid at room temperature and at atmospheric pressure.

The liquid fatty substances of the invention preferably have a viscosity of less than or equal to 2 Pa.s, better still less than or equal to 1 Pa.s and even better still less than or equal to 0.1 Pa.s at a temperature of 25°C and at a shear rate of 1 s⁻¹.

The liquid fatty substances of the invention may be non-silicone, i.e. they do not comprise in their structure any -Si-O-Si- sequences.

In one variant of the invention, the liquid fatty substance(s) that may be used in the composition according to the invention may be chosen from liquid non-silicone fatty substances.

The non-silicone liquid fatty substances that may be used in the composition according to the invention are chosen from hydrocarbons, fatty alcohols, fatty acid and/or fatty alcohol esters, non-salified fatty acids and fatty-chain alkoxysilanes.

The non-silicone liquid fatty substances generally have in their structure a hydrocarbon-based chain comprising at least 6 carbon atoms.

The term "liquid hydrocarbon" means a hydrocarbon composed solely of carbon and hydrogen atoms, which is liquid at standard temperature (25°C) and at atmospheric pressure (760 mmHg, i.e. 1.013 × 10⁵ Pa).

More particularly, the liquid hydrocarbons that may be used in the composition according to the invention are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane,
- linear or branched hydrocarbons of synthetic, animal or mineral origin, with more than 16 carbon atoms, such as liquid paraffin or liquid petrolatum, polydecenes, hydrogenated polyisobutene such as that sold under the brand name Parleam® by the company NOF Corporation, and squalane.

Preferably, the liquid hydrocarbon(s) are chosen from liquid paraffins, isoparaffins, liquid petroleum jelly, undecane, tridecane and isododecane, and mixtures thereof.

In a most particularly preferred variant, the liquid hydrocarbon(s) are chosen from liquid paraffin, liquid petroleum jelly, isoparaffins, isododecane and a mixture of undecane and tridecane.

The term "liquid fatty alcohol" means a non-glycerolated and non-oxyalkylenated fatty alcohol, which is liquid at standard temperature (25°C) and at atmospheric pressure (760 mmHg, i.e. 1.013 × 10⁵ Pa). Preferably, the liquid fatty alcohols that may be used in the composition according to the invention comprise from 8 to 30 carbon atoms and they may be saturated or unsaturated.

The saturated liquid fatty alcohols are preferably branched. They may optionally comprise in their structure at least one aromatic or non-aromatic ring. They are preferably acyclic.

More particularly, the saturated liquid fatty alcohols that may be used in the composition according to the invention are chosen from octyldodecanol, 2-decyltetradecanol, isostearyl alcohol and 2-hexyldecanol.

Octyldodecanol and 2-decyltetradecanol are most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. They are preferably acyclic.

More particularly, the unsaturated liquid fatty alcohols that may be used in the composition according to the invention are chosen from oleyl alcohol, linoleyl alcohol, linolenyl alcohol and undecylenyl alcohol.

Oleyl alcohol is most particularly preferred.

The term "liquid fatty ester" means an ester derived from a fatty acid and/or from a fatty alcohol that is liquid at standard temperature (25°C) and at atmospheric pressure (760 mmHg, i.e. 1.013 × 10⁵ Pa).

More particularly, the liquid esters are chosen from saturated or unsaturated, linear or branched C₁-C₂₆ aliphatic mono- or polyacids and of saturated or unsaturated, linear or branched C₁C₂₆ aliphatic mono- or polyalcohols, the total number of carbon atoms in the esters being greater than or equal to 10.

Preferably, for the esters of monoalcohols, at least one of the alcohol or of the acid from which the esters of the invention result is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may be used.

Mention may be made in particular of diethyl sebacate, diisopropyl sebacate, diisopropyl adipate, di-n-propyl adipate, dioctyl adipate, diisostearyl adipate, dioctyl maleate, glyceryl undecylenate, octyldodecyl stearoyl stearate, pentaerythrityl monoricinoleate, pentaerythrityl tetraisononanoate, pentaerythrityl tetrapelargonate, pentaerythrityl tetraisostearate, pentaerythrityl tetraoctanoate, propylene glycol dicaprylate, propylene glycol dicaprate, tridecyl erucate, triisopropyl citrate, triisostearyl citrate, glyceryl trilactate, glyceryl trioctanoate, trioctyldodecyl citrate, trioleyl citrate, propylene glycol dioctanoate, neopentyl glycol diheptanoate, diethylene glycol diisononanoate and polyethylene glycol distearates.

Among the esters mentioned above, use is preferentially made of ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, propylene glycol dicaprylate, butyl stearate, isobutyl stearate; dioctyl malate, hexyl laurate, 2-hexyldecyl laurate, isononyl isononanoate or cetyl octanoate.

Among the liquid fatty esters, use may be made of sugar esters and diesters of C₆-C₃₀ and preferably C₁₂-C₂₂ fatty acids.

The term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Preferably, the said sugars are chosen from saccharose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids.

If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

The said esters may be chosen, for example, from oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and especially of sucrose, glucose or methylglucose mono- or dioleates, stearates, behenates, oleopalmitates, linoleates, linolenates or oleostearates, or alternatively of methylglucose dioleate (Glucate® DO).

Among the sugar esters, use may be made of pentaerythrityl esters, preferably pentaerythrityl tetraisostearate, pentaerythrityl tetraoctanoate, and caprylic and capric hexaesters as a mixture with dipentaerythritol.

Among the natural or synthetic monoacid, diacid or triacid esters of glycerol, use may be made of plant oils or synthetic oils.

More particularly, the said plant oil(s) or synthetic oil(s) are chosen from triglyceride oils of plant or synthetic origin, such as liquid fatty acid triglycerides containing from 6 to 30 carbon atoms, for instance heptanoic or octanoic acid triglycerides, or alternatively, for example, sesame oil, soybean oil, coffee oil, safflower oil, borage oil, sunflower oil, olive oil, apricot kernel oil, camellia oil, bambara pea oil, avocado oil, mango oil, rice bran oil, cotton seed oil, rose oil, kiwi seed oil, sea buckthorn pulp oil, blueberry seed oil, poppy seed oil, orange pip oil, sweet almond oil, palm oil, coconut oil, vernonia oil, marjoram oil, baobab oil, rapeseed oil, ximenia oil, pracaxi oil, caprylic/capric acid triglycerides such as those sold by the company Stéarineries Dubois or those sold under the names Miglyol® 810, 812 and 818 by the company Dynamit Nobel, jojoba oil and shea butter oil.

As liquid esters that may be used according to the invention, use is preferably made of triglycerides of plant origin, in particular oils chosen from avocado oil, olive oil, camellia oil and apricot kernel oil, and mixtures thereof, and C₄-C₂₂ dicarboxylic or tricarboxylic acid esters of C₁-C₂₂ alcohols, in particular 1,3-propanediol dicaprylate.

The term "fatty acid" means a non-salified fatty acid, i.e. the fatty acid must not be in the form of a generally soluble soap, i.e. it must not be salified with a base.

More particularly, the liquid fatty acids according to the invention are chosen from the acids of formula RCOOH, in which R is a saturated or unsaturated, linear or branched radical preferably comprising from 7 to 39 carbon atoms.

Preferably, R is a C₇-C₂₉ alkyl or C₇-C₂₉ alkenyl group and better still a C₁₂-C₂₄ alkyl or C₁₂-C₂₄ alkenyl group. R may be substituted with one or more hydroxyl groups and/or one or more carboxyl groups.

Preferentially, the liquid fatty acid(s) are chosen from oleic acid, linoleic acid and isostearic acid.

The term "alkoxysilanes" means alkoxysilanes bearing a fatty chain preferentially comprising 16 or 18 carbon atoms.

Even more preferentially, the alkoxysilanes may be chosen from hexadecyltriethoxysilane and octadecyltriethoxysilane.

Preferentially, the liquid non-silicone fatty substance(s) that may be used in the composition according to the invention are chosen from hydrocarbons, in particular linear or branched C₆-C₁₆ alkanes and linear or branched hydrocarbons of mineral, animal or synthetic origin, of more than 16 carbon atoms, such as liquid paraffins and derivatives thereof, petroleum jelly, liquid petroleum jelly; fatty acid esters, in particular oils of plant origin and C₄-C₂₂ dicarboxylic or tricarboxylic acid esters of C₁-C₂₂ alcohols, these esters being chosen more preferentially from triglycerides of plant origin, and liquid fatty alcohols, and mixtures thereof.

More preferentially, the non-silicone liquid fatty substance(s) are chosen from liquid petroleum jelly, isoparaffins, isododecane, undecane, tridecane, avocado oil, olive oil, camellia oil, apricot kernel oil, oleic acid and 1,3-propanediol dicaprylate, and mixtures thereof.

Even more preferentially, the non-silicone liquid fatty substance(s) are chosen from avocado oil, liquid petroleum jelly and oleic acid, and a mixture thereof.

In another variant, the liquid fatty substance(s) that may be used in the composition according to the invention may be chosen from silicones.

Preferably, the liquid silicone(s) are chosen from polydialkylsiloxanes, in particular polydimethylsiloxanes (PDMSs), and organomodified polysiloxanes comprising at least one functional group chosen from amino groups, aryl groups and alkoxy groups.

Organopolysiloxanes are defined in greater detail in Walter Noll's Chemistry and Technology of Silicones (1968), Academic Press. They may be volatile or non-volatile.

The volatile silicones are more particularly chosen from silicones with a boiling point of between 60°C and 260°C, and even more particularly silicones chosen from: (i) cyclic polydialkylsiloxanes comprising from 3 to 7 and preferably from 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, and Silbione® 70045 V5 by Rhodia, and mixtures thereof.

Mention may also be made of cyclocopolymers of the dimethylsiloxane/methylalkylsiloxane type, such as Volatile Silicone® FZ 3109 sold by the company Union Carbide, of formula:

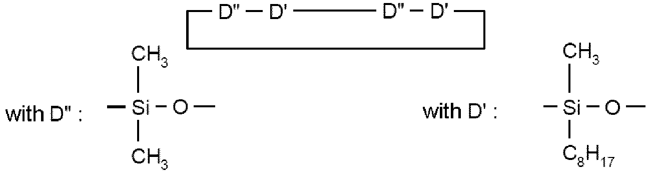

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane; (ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.*

The liquid non-volatile silicones that may be used in the composition according to the invention may preferably be liquid non-volatile polydialkylsiloxanes, polyorganosiloxanes modified with organofunctional groups chosen from amine groups, aryl groups and alkoxy groups, and also mixtures thereof.

These silicones are more particularly chosen from polydialkylsiloxanes, among which mention may be made mainly of polydimethylsiloxanes bearing trimethylsilyl end groups. The viscosity of the silicones is measured at 25°C according to ASTM Standard 445 Appendix C.

Among these polydialkylsiloxanes, mention may be made, in a nonlimiting manner, of the following commercial products:
- the Silbione® oils of the 47 and 70 047 series or the Mirasil® oils sold by Rhodia;
- the oils of the Mirasil® series sold by the company Rhodia;
- the oils of the 200 series from the company Dow Corning;
- the Viscasil® oils from General Electric and certain oils of the SF series (SF 96, SF 18) from General Electric.

Mention may also be made of polydimethylsiloxanes bearing dimethylsilanol end groups known under the name dimethiconol (CTFA), such as the oils of the 48 series from the company Rhodia.

The organomodified silicones that may be used in accordance with the invention are silicones as defined previously and comprising in their structure one or more organofunctional groups attached via a hydrocarbon-based group.

The organomodified silicones may be polydiarylsiloxanes, in particular polydiphenylsiloxanes, and polyalkylarylsiloxanes functionalized with the organofunctional groups mentioned previously.

The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.

Among these polyalkylarylsiloxanes, examples that may be mentioned include the products sold under the following names:
- the Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Particularly preferred polyalkylarylsiloxanes are phenyl trimethicone according to INCI (CTFA) nomencalature.

Among the organomodified silicones, mention may also be made of polyorganosiloxanes comprising:
- substituted or unsubstituted amine groups, for instance the products sold under the names GP 4 Silicone Fluid and GP 7100 by the company Genesee. The substituted amine groups are, in particular, C₁-C₄ aminoalkyl groups;
- alkoxylated groups, such as the product sold under the name Silicone Copolymer F-755 by SWS Silicones and Abil Wax® 2428, 2434 and 2440 by the company Goldschmidt.

Preferably, the compositions according to the invention comprise at least one non-silicone liquid fatty substance. Even more preferentially, the compositions according to the invention comprise one or more liquid fatty substances chosen from hydrocarbons, in particular liquid paraffin or liquid petroleum jelly.

According to another preferred embodiment, the composition according to the invention comprises at least one silicone liquid fatty substance, preferably chosen from organomodified silicones, especially polydiarylsiloxanes and polyalkylarylsiloxanes functionalized with organofunctional groups, and preferably phenyl trimethicone.

When one or more liquid fatty substances are present, the content preferably ranges from 0.01% to 20.0% by weight, better still from 0.1% to 15.0% by weight and even better still from 2.0% to 10.0% by weight relative to the total weight of the composition.

According to a preferred embodiment, the composition according to the invention comprises at least 5.0% by weight of at least one non-liquid fatty substance and at least 2.0% by weight of at least one liquid fatty substance.

According to this embodiment, the liquid fatty substance is chosen from hydrocarbons, preferably liquid paraffin or liquid petroleum jelly.

According to a preferred embodiment, the nonionic surfactant(s) are different from the non-liquid fatty substance(s) previously described.

The composition according to the invention may also optionally comprise one or more cationic polymers.

The term "cationic polymer" means any polymer comprising cationic groups and/or groups that can be ionized to cationic groups. Preferably, the cationic polymer is hydrophilic or amphiphilic. The preferred cationic polymers are chosen from those that contain units comprising primary, secondary, tertiary and/or quaternary amine groups that may either form part of the main polymer chain or may be borne by a side substituent directly connected thereto.

The cationic polymers that may be used preferably have a weight-average molar mass (Mw) of between 500 and 5×10⁶ approximately and preferably between 10³ and 3×10⁶ approximately.

Among the cationic polymers, mention may be made more particularly of:
(1) homopolymers or copolymers derived from acrylic or methacrylic esters or amides and comprising at least one of the units of the following formulae: 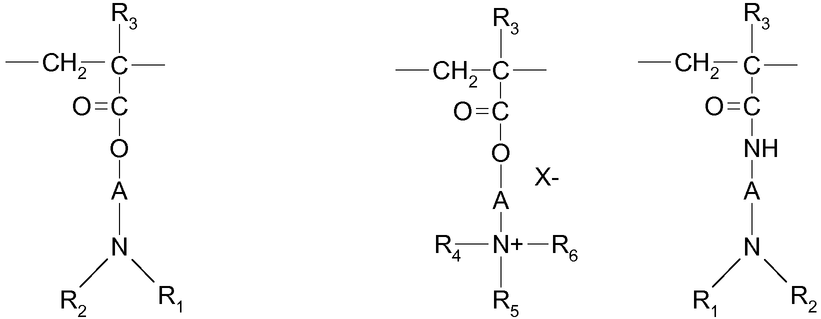 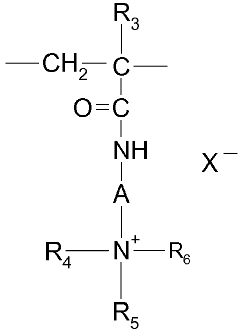 in which:
   - R3, which may be identical or different, denote a hydrogen atom or a CH₃ radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R4, R5 and R6, which may be identical or different, represent an alkyl group containing from 1 to 18 carbon atoms or a benzyl radical, preferably an alkyl group containing from 1 to 6 carbon atoms;
   - R1 and R2, which may be identical or different, represent a hydrogen atom or an alkyl group containing from 1 to 6 carbon atoms, preferably methyl or ethyl;
   - X denotes an anion derived from a mineral or organic acid, such as a methosulfate anion or a halide such as chloride or bromide.

   The copolymers of family (1) may also contain one or more units derived from comonomers that may be selected from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters.
   Among these copolymers of family (1), mention may be made of:
   - copolymers of acrylamide and of dimethylaminoethyl methacrylate quaternized with dimethyl sulfate or with a dimethyl halide, such as the product sold under the name Hercofloc by the company Hercules,
   - copolymers of acrylamide and of methacryloyloxyethyltrimethylammonium chloride, such as those sold under the name Bina Quat P 100 by the company Ciba Geigy,
   - copolymers of acrylamide and of methacryloyloxyethyltrimethylammonium methosulfate, such as the product sold under the name Reten by the company Hercules,
   - quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers, such as those sold under the name Styleze CC 10 by ISP,
   - quaternized vinylpyrrolidone/dimethylaminopropylmethacrylamide copolymers such as the product sold under the name Gafquat HS 100 by the company ISP,
   - preferably crosslinked polymers of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, more particularly methylenebisacrylamide. A crosslinked acrylamide/methacryloyloxyethyltrimethylammonium chloride copolymer (20/80 by weight) in the form of a dispersion containing 50% by weight of the said copolymer in mineral oil may be used more particularly. This dispersion is sold under the name Salcare® SC 92 by the company Ciba. A crosslinked methacryloyloxyethyltrimethylammonium chloride homopolymer containing about 50% by weight of the homopolymer in mineral oil or in a liquid ester can also be used. These dispersions are sold under the names Salcare® SC 95 and Salcare® SC 96 by the company Ciba.
(2) Cationic polysaccharides, especially cationic celluloses and galactomannan gums. Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.
   The cellulose ether derivatives comprising quaternary ammonium groups are especially described in French patent 1 492 597, and mention may be made of the polymers sold under the name Ucare Polymer "JR" (JR 400 LT, JR 125 and JR 30M) or "LR" (LR 400 or LR 30M) by the company Amerchol. These polymers are also defined in the CTFA dictionary as quaternary ammoniums of hydroxyethylcellulose that have reacted with an epoxide substituted with a trimethylammonium group.
   Cationic cellulose copolymers or cellulose derivatives grafted with a water-soluble quaternary ammonium monomer are described especially in US patent 4 131 576, and mention may be made of hydroxyalkylcelluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.
   The cationic galactomannan gums are described more particularly in US patents 3 589 578 and 4 031 307, and mention may be made of guar gums comprising cationic trialkylammonium groups. Use is made, for example, of guar gums modified with a 2,3-epoxypropyltrimethylammonium salt (for example, chloride). Such products are especially sold under the names Jaguar C13 S, Jaguar C 15, Jaguar C 17 or Jaguar C162 by the company Rhodia.
(3) Polymers formed from piperazinyl units and divalent alkylene or hydroxyalkylene radicals containing straight or branched chains, optionally interrupted with oxygen, sulfur or nitrogen atoms or with aromatic or heterocyclic rings, and also the oxidation and/or quaternization products of these polymers.
(4) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they comprise one or more tertiary amine functions, they can be quaternized.
(5) Polyamino amide derivatives resulting from the condensation of polyalkylene polyamines with polycarboxylic acids followed by alkylation with bifunctional agents. Mention may be made, for example, of adipic acid/dialkylaminohydroxyalkyldialkylenetriamine polymers in which the alkyl radical comprises from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz.
(6) Polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyamino amide being reacted with epichlorohydrin in a mole ratio of epichlorohydrin relative to the secondary amine group of the polyamino amide preferably of between 0.5:1 and 1.8:1. Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.
(7) Cyclopolymers of alkyldiallylamine or of dialkyldiallylammonium, such as the homopolymers or copolymers containing, as main constituent of the chain, units corresponding to formula (I) or (II): 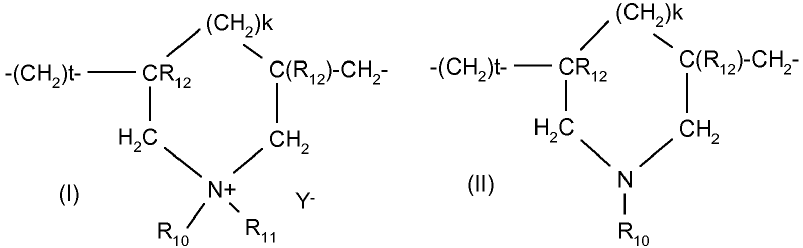 in which:
   - k and t are equal to 0 or 1, the sum k + t being equal to 1;
   - R12 denotes a hydrogen atom or a methyl radical;
   - R10 and R11, independently of each other, denote a C1-C6 alkyl group, a hydroxyl(C1-C5)alkyl group, a C1-C4 amidoalkyl group; or alternatively R10 and R11 may denote, together with the nitrogen atom to which they are attached, an heterocyclic group such as piperidinyl or morpholinyl; R10 and R11, independently of each other, preferably denote a C1-C4 alkyl group;
   - Y⁻ is an anion such as bromide, chloride, acetate, borate, citrate, tartrate, bisulfate, bisulfite, sulfate or phosphate.

   Mention may be made more particularly of the dimethyldiallylammonium salt (for example chloride) homopolymer sold for example under the name Merquat 100 by the company Nalco, and the copolymers of diallyldimethylammonium salts (for example chloride) and of acrylamide, sold especially under the name Merquat 550 or Merquat 7SPR.
(8) Quaternary diammonium polymers comprising repeating units of formula: 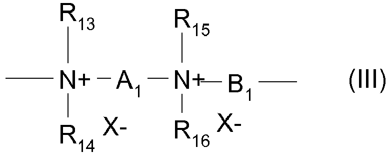 in which:
   - R13, R14, R15 and R16, which may be identical or different, represent aliphatic, alicyclic or arylaliphatic radicals comprising from 1 to 20 carbon atoms, or C1-C12 hydroxyalkylaliphatic radicals,
      or else R13, R14, R15 and R16, together or separately, constitute, with the nitrogen atoms to which they are attached, heterocycles optionally comprising a second non-nitrogen heteroatom,
      or else R13, R14, R15 and R16 represent a linear or branched C₁-C₆ alkyl radical substituted with a nitrile, ester, acyl, amide or -CO-O-R17-D or -CO-NH-R17-D group in which R17 is an alkylene and D is a quaternary ammonium group;
   - A1 and B1 represent divalent polymethylene groups comprising from 2 to 20 carbon atoms, linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
   - X⁻ denotes an anion derived from a mineral or organic acid;
   it being understood that A1, R13 and R15 can form, with the two nitrogen atoms to which they are attached, a piperazine ring;
   in addition, if A1 denotes a linear or branched, saturated or unsaturated alkylene or hydroxyalkylene radical, B1 may also denote a group (CH₂)ₙ-CO-D-OC-(CH₂)ₙ- in which D denotes:
   a) a glycol residue of formula -O-Z-O-, in which Z denotes a linear or branched hydrocarbon-based radical, or a group corresponding to one of the following formulae: -(CH₂-CH₂-O)ₓ-CH₂-CH₂- and -[CH₂-CH(CH₃)-O]_{y}-CH₂-CH(CH₃)-, where x and y denote an integer from 1 to 4, representing a defined and unique degree of polymerization or any number from 1 to 4 representing an average degree of polymerization;
   b) a bis-secondary diamine residue such as a piperazine derivative;
   c) a bis-primary diamine residue of formula: -NH-Y-NH-, where Y denotes a linear or branched hydrocarbon-based radical, or else the divalent radical -CH₂-CH₂-S-S-CH₂-CH₂-;
   d) a ureylene group of formula: -NH-CO-NH-;

Preferably, X⁻ is an anion such as chloride or bromide. These polymers have a number-average molar mass (Mn) generally of between 1000 and 100 000.

Mention may be made more particularly of polymers that are composed of repeating units corresponding to the formula: 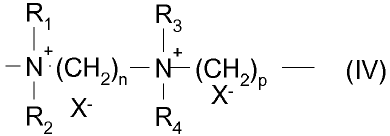 in which R1, R2, R3 and R4, which may be identical or different, denote an alkyl or hydroxyalkyl radical containing from 1 to 4 carbon atoms, n and p are integers ranging from 2 to 20, and X⁻ is an anion derived from an organic or mineral acid.

A particularly preferred compound of formula (IV) is that for which R1, R2, R3 and R4 represent a methyl radical and n = 3, p = 6 and X = CI, known as Hexadimethrine chloride according to the INCI (CTFA) nomenclature.
(9) Polyquaternary ammonium polymers comprising units of formula (V): 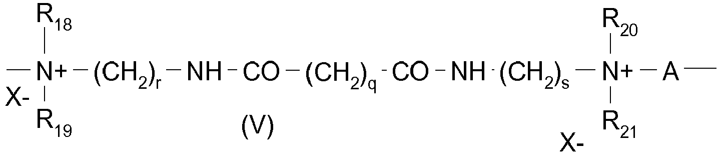 in which:
   - R18, R19, R20 and R21, which may be identical or different, represent a hydrogen atom or a methyl, ethyl, propyl, β-hydroxyethyl, β-hydroxypropyl or -CH₂CH₂(OCH₂CH₂)ₚOH group, in which p is equal to 0 or to an integer between 1 and 6, with the proviso that R18, R19, R20 and R21 do not simultaneously represent a hydrogen atom,
   - r and s, which may be identical or different, are integers between 1 and 6,
   - q is equal to 0 or to an integer between 1 and 34,
   - X- denotes an anion such as a halide,
   - A denotes a dihalide radical or preferably represents -CH₂-CH₂O-CH₂-CH₂-.

   Examples that may be mentioned include the products Mirapol® A15, Mirapol® AD1, Mirapol® AZ1 and Mirapol® 175 sold by the company Miranol.
(10) Quaternary polymers of vinylpyrrolidone and of vinylimidazole, for instance the products sold under the names Luviquat® FC 905, FC 550 and FC 370 by the company BASF.
(11) Polyamines such as Polyquart® H sold by Cognis, referred to under the name Polyethylene glycol (15) tallow polyamine in the CTFA dictionary.
(12) Polymers comprising in their structure:
   (a) one or more units corresponding to formula (A) below: 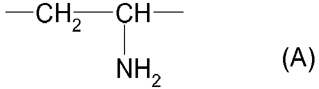
   (b) optionally, one or more units corresponding to formula (B) below:

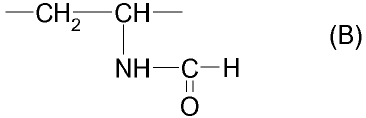

In other words, these polymers may be chosen especially from homopolymers or copolymers comprising one or more units derived from vinylamine and optionally one or more units derived from vinylformamide.

Preferably, these cationic polymers are chosen from polymers comprising, in their structure, from 5 mol% to 100 mol% of units corresponding to formula (A) and from 0 to 95 mol% of units corresponding to formula (B), preferentially from 10 mol% to 100 mol% of units corresponding to formula (A) and from 0 to 90 mol% of units corresponding to formula (B).

These polymers may be obtained, for example, by partial hydrolysis of polyvinylformamide. This hydrolysis may be performed in an acidic or basic medium.

The weight-average molecular mass of the said polymer, measured by light scattering, may range from 1000 to 3 000 000 g/mol, preferably from 10 000 to 1 000 000 g/mol and more particularly from 100 000 to 500 000 g/mol.

The polymers comprising units of formula (A) and optionally units of formula (B) are sold especially under the name Lupamin by the company BASF, for instance, and in a non-limiting manner, the products sold under the names Lupamin 9095, Lupamin 5095, Lupamin 1095, Lupamin 9030 (or Luviquat 9030) and Lupamin 9010.

Other cationic polymers that may be used in the context of the invention are cationic proteins or cationic protein hydrolysates, polyalkyleneimines, in particular polyethyleneimines, polymers comprising vinylpyridine or vinylpyridinium units, condensates of polyamines and of epichlorohydrin, quaternary polyureylenes and chitin derivatives.

Preferably, the cationic polymers are chosen from the polymers of families (1), (2), (7) and (10) mentioned above.

Among the cationic polymers mentioned above, the ones that may preferably be used are cationic polysaccharides, especially cationic celluloses and galactomannan gums, and in particular quaternary cellulose ether derivatives such as the products sold under the name "JR 400" by the company Amerchol, cationic cyclopolymers, in particular dimethyldiallylammonium salt (for example chloride) homopolymers or copolymers, sold under the names Merquat 100, Merquat 550 and Merquat S by the company Nalco, quaternary polymers of vinylpyrrolidone and of vinylimidazole, optionally crosslinked homopolymers or copolymers of methacryloyloxy(C₁-C₄)alkyltri(C₁-C₄)alkylammonium salts, and mixtures thereof.

When one or more cationic polymers are present, the content preferably ranges from 0.01% to 10.0% by weight, better still from 0.1% to 5.0% by weight and even better still from 0.5% to 3.0% by weight relative to the total weight of the composition.

The composition of the invention generally comprises water or a mixture of water and one or more organic solvents.

Among the suitable organic solvents, mention may be made more particularly of non-aromatic monoalcohols such as ethyl alcohol, isopropyl alcohol, or polyols or polyol ethers, for instance ethylene glycol monomethyl, monoethyl or monobutyl ether, propylene glycol or ethers thereof, for instance propylene glycol monomethyl ether, butylene glycol, dipropylene glycol, and also diethylene glycol alkyl ethers, for instance diethylene glycol monoethyl ether or monobutyl ether, or alternatively polyols such as glycerol. Polyethylene glycols and polypropylene glycols, and mixtures of all these compounds, can also be used as solvent.

The water and the organic solvents described above, if they are present, usually represent from 0.1% to 15% by weight and more preferentially from 0.5% to 5% by weight relative to the total weight of the composition.

The pH of the composition according to the invention generally ranges from 1.5 to 14 and preferably from 7 to 12.7. It may be adjusted by adding either alkaline agents such as aqueous ammonia, monoethanolamine, diethanolamine, triethanolamine, isopropanolamine, 2-methyl-2-amino-1-propanol, 1,3-propanediamine, guanidine, arginine, an alkali metal or ammonium carbonate or bicarbonate, an organic carbonate such as guanidine carbonate, or alternatively an alkali metal hydroxide, or acidifying agents such as hydrochloric acid, acetic acid, lactic acid, boric acid, citric acid and phosphoric acid.

Preferably, the composition according to the invention comprises at least one alkaline agent.

In particular, the composition may comprise aqueous ammonia, ammonium bicarbonate or one or more alkanolamines, especially monoethanolamine.

The composition in accordance with the invention may also comprise one or more additional cosmetic agents.

The additional cosmetic agent(s) may be chosen from anionic, nonionic, amphoteric and zwitterionic polymers or mixtures thereof; anionic surfactants other than the phosphoric surfactants of the invention, especially sulfate, sulfonate or carboxylate surfactants, amphoteric surfactants or cationic surfactants; pigments; thickeners; antioxidants; penetrants; sequestrants; fragrances; buffers; dispersants; reduction regulators such as dithio acids, such as dithiodiglycolic acid and salts thereof, film-forming agents; preserving agents; stabilizers; opacifiers; fragrances.

Needless to say, a person skilled in the art will take care to select this or these optional additional compound(s) such that the advantageous properties intrinsically associated with the composition in accordance with the invention are not, or are not substantially, adversely affected by the envisaged addition(s).

The above additional cosmetic agents are generally present in an amount, for each of them, of between 0 and 20% by weight relative to the total weight of the composition.

Preferably, the composition may comprise one or more thickeners, which may be chosen from natural or synthetic, anionic, amphoteric, zwitterionic, nonionic or cationic, associative or non-associative polymeric thickeners, and non-polymeric thickeners.

Examples of polymeric thickeners that are mentioned include cellulose-based thickeners, for example hydroxyethylcellulose, hydroxypropylcellulose and carboxymethylcellulose, guar gum and derivatives thereof, for example hydroxypropyl guar, sold by the company Rhodia under the reference Jaguar HP 105, gums of microbial origin, such as xanthan gum and scleroglucan gum, synthetic polymeric thickeners, such as crosslinked homopolymers of acrylic acid or of acrylamidopropanesulfonic acid, for example Carbomer, or nonionic, anionic or amphoteric associative polymers, such as the polymers sold under the names Pemulen TR1 or TR2 by the company Goodrich, Salcare SC90 by the company Allied Colloids, Aculyn 22, 28, 33, 44 or 46 by the company Rohm & Haas and Elfacos T210 and T212 by the company Akzo.

Preferably, the composition is in the form of a cream.

The composition according to the invention may advantageously have a viscosity at 25°C ranging from 2 to 20 Pa.s, preferentially ranging from 2 to 15 Pa.s and even more preferentially ranging from 2.5 to 10 Pa.s.

The viscosity of the composition is determined at 25°C using a Rheomat 180 rotary viscometer (Jean LAMY SA) equipped with an anchor/cup type geometer spindle 3 (MS-r 3) or spindle 4 (MS-r4) with a spin speed of 200 rpm and use of the calibration curves provided by the viscometer supplier.

The present invention also relates to a process for permanently reshaping, especially for straightening or perming, keratin fibres, in particular human keratin fibres such as the hair, which consists in performing the following steps:
(i) a composition according to the invention is applied to the said fibres and is left to stand on the fibres for a time sufficient for shaping, and
(ii) an oxidizing composition is optionally applied to the said fibres for a time sufficient for fixing the shape.

The leave-on time after application of the composition according to the invention generally ranges from 5 to 60 minutes, particularly from 5 to 45 minutes and preferably from 10 to 45 minutes.

The oxidizing composition used in step (ii) of the permanent reshaping process conventionally comprises one or more oxidizing agents, in general aqueous hydrogen peroxide solution, an alkali metal bromate, a persalt or a polythionate, and even more preferentially aqueous hydrogen peroxide solution.

The pH of the oxidizing composition generally ranges from 2 to 10.

The leave-on time of the oxidizing composition generally ranges from 2 to 30 minutes and preferably from 5 to 15 minutes.

In particular, the composition according to the invention is applied to reduce the disulfide bonds of keratin, the keratin fibres being placed under mechanical tension before, during or after the said application.

When it is desired to perform perming, mechanical means are preferably used, such as curlers, in order to place the keratin fibres under tension, the composition according to the invention being applied before, during or after the hair-shaping means, preferably before.

The composition in accordance with the present invention may be applied to wet hair that has been rolled up beforehand on rollers that are from 2 to 30 mm in diameter. The composition according to the invention may also be applied gradually as the hair is rolled up. Generally, the composition according to the invention is then left to act for a time of from 5 to 60 minutes.

After applying the composition according to the invention, the head of hair may also be subjected to a heat treatment by heating to a temperature of between 30 and 250°C throughout all or part of the leave-on time. In practice, this operation may be performed using a hairstyling hood, a hairdryer, a round or flat iron, an infrared ray dispenser or other standard heating appliances.

It is especially possible to use, both as a heating means and as a means for shaping the head of hair, an iron that heats to a temperature of between 60 and 230°C and preferably between 120 and 230°C, the use of the heating iron taking place after the intermediate rinsing step following the application of the composition according to the invention.

The curler itself may be a heating means.

The oxidizing composition for reforming the disulfide bonds of keratin is then applied to the rolled up or unrolled hair, generally for a leave-on time of 2 to 15 minutes.

In the context of a hair straightening or relaxing process, the composition according to the invention is applied to the hair, and the hair is then subjected to mechanical reshaping for fixing the hair in its new shape, by means of a hair straightening operation, with a large-toothed comb, with the back of a comb, by hand or with a brush. A leave-on time of from 5 to 60 minutes is generally implemented.

This application may also be followed by a heating treatment, especially using an iron.

The straightening of the hair may also be performed, totally or partly, using a heating iron at between 60 and 230°C and preferably between 120 and 230°C.

The oxidizing composition as defined above is then optionally applied, and is generally left to act for about 2 to 15 minutes, and the hair is then optionally rinsed thoroughly, generally with water.

After performing the permanent reshaping process, the keratin fibres are optionally rinsed.

Preferably, the keratin fibres impregnated with the oxidizing composition are rinsed thoroughly with water. The keratin fibres may optionally be separated, before or after, from the means needed to keep them under tension.

The keratin fibres may then be washed with a shampoo, rinsed and dried or left to dry.

Preferably, the permanent reshaping process is a process for perming keratin fibres, in particular human keratin fibres such as the hair.

The composition according to the invention may also be used for hair removal.

The following examples serve to illustrate the invention without, however, exhibiting a limiting nature.

### Examples of compositions for permanently reshaping the hair

Compositions A to D according to the invention and comparative composition E were prepared from the ingredients given in the table below, in grams of active material:

| **INCI name** | **A (inv)** | **B (inv)** | **C (inv)** | **D (inv)** | **E (comp)** |
|---|---|---|---|---|---|
| Ammonium bicarbonate | **-** | **-** | 2.15 | - | |
| Diethylenetriaminepentaacetic acid, pentasodium salt | 0.16 | 0.16 | 0.16 | 0.16 | 0.16 |
| Aminomethylpropanol | - | - | - | 1 | - |
| Aqueous ammonia expressed as NH₃ | 0.72 | 0.72 | - | - | 0.72 |
| Diammonium dithiodiglycolate, | 3.6 | 3.6 | 2.4 | - | 3.6 |
| Ethanolamine | 1.5 | 1.5 | 2.5 | 2.3 | 1.5 |
| Ammonium thioglycolate | 11.78 | 11.78 | 8.16 | - | 11.78 |
| Cysteine | - | - | - | 4 | - |
| Mineral oil | 3 | 3 | 3 | 3 | - |
| Cetearyl alcohol | 7 | 7 | 7 | 7 | 8 |
| Fragrance | - | 0.6 | 0.6 | - | - |
| Polydimethyldiallylammonium chloride | - | 1 | 1 | 1 | - |
| PPG-5-Ceteth-20 | 3 | 3 | 3 | 3 | - |
| Mixture of cetearyl alcohol, dicetyl phosphate and oxyethylenated cetyl phosphate (10 OE) (75/14/11) (*Crodafos CES-PA from Croda*) | 5 | 5 | 5 | 5 | |
| Poly[(dimethylimino)-1,3-propanediyl(dimethylimino)-1,6-hexanediyl dichloride] | - | 0.6 | - | - | - |
| Ceteth-2 | - | - | - | - | 3 |
| Behentrimonium chloride | - | - | - | - | 3.22 |
| Cetrimonium chloride | | | | | 1 |
| Cetyl palmitate | - | - | - | - | 2 |
| Water | qs 100 | qs 100 | qs 100 | qs 100 | qs 100 |

### 1. According to a first standard straightening process (cold straightening)

160 to 200 g, depending on the head of hair, of compositions A, B, C or D were applied to prewashed curly and/or voluminous wet hair, lock by lock from the roots to the ends.

The compositions were left to stand on the hairs for a time depending on the degree of sensitization of the hair, namely between 10 and 50 minutes.

The hairs were rinsed and then dried manually with a towel.

An oxidizing composition comprising aqueous hydrogen peroxide solution (8 volumes) was then applied to the entire head of hair and left to act for 10 minutes.

The hair was then dried either under a hood or using a hairdryer with blow-drying followed by an application of flat tongs, or alternatively in the open air.

The hairs are straight and smooth with compositions A, B and C, the straightening performance qualities being similar to those for composition E.

Moreover, during the application of the compositions, markedly less odour is noted with the compositions according to the invention when compared with composition E.

In particular, the evolution of ammonia during the leave-on time of the compositions on the hair was measured by chemiluminescence. For composition A according to the invention, an evolution of NH₃ of 2821 ppm over 100 s was measured. For comparative composition E, an evolution of NH₃ of 8866 ppm over 100 s was measured. For an identical content of ammonium thioglycolate, a significant reduction of the ammonia odour is thus noted with the composition according to the invention.

### 2. According to a second straightening process (Japanese straightening)

160 to 200 g, depending on the head of hair, of compositions A, B or C were applied to prewashed curly and/or voluminous wet hair, lock by lock from the roots to the ends.

The compositions were left to stand on the hairs for a time depending on the degree of sensitization of the hair, namely between 15 and 45 minutes.

The hairs were rinsed with warm water and then dried manually with a towel.

The hairs were then 90% predried using a hairdryer.

A straightening iron was then passed at a temperature of 190 or 210°C through very fine locks of hair, the iron being passed three times through each lock.

An oxidizing composition in the form of a cream comprising aqueous hydrogen peroxide solution (8 volumes) was then applied to the entire head of hair and left to act for 10 minutes, followed by rinsing with warm water.

Little evolution of ammonia or volatile sulfureous compounds is noted. In addition, the head of hair is straight.

### 3. According to a process for permanently reshaping the hair forming curls (Digital Perm)

160 to 200 g, depending on the head of hair, of compositions A, B or C were applied to prewashed straight wet hair, lock by lock from the roots to the ends.

The compositions were left to stand on the hairs for a time depending on the degree of sensitization of the hair, namely between 15 and 45 minutes.

The hairs were rinsed with warm water and then dried manually with a towel.

The hairs were then rolled up on heating curlers. The hairs were then heated at 50°C for about 20 to 30 minutes. At the end of the heating time, the curlers were cooled with a cold hairdryer.

An oxidizing composition in liquid form comprising aqueous hydrogen peroxide solution (8 volumes) was then applied to the entire head of hair and left to act for 10 minutes.

The hair was then unrolled from the curlers, followed by rinsing with warm water.

Little evolution of ammonia or volatile sulfureous compounds is noted. In addition, a curly head of hair is obtained.

### 4. According to a semi-durable straightening process:

100 to 160 g, depending on the head of hair, of composition D were applied to unmanageable prewashed curly and/or voluminous wet hair, lock by lock from the roots to the ends.

The compositions were left to stand on the hairs for a time depending on the degree of sensitization of the hair, namely between 10 and 20 minutes.

The hairs were rinsed and then dried manually with a towel.

A leave-in treatment product was applied to the hairs and the hairs were then dried by blow-drying, optionally followed by treating with flat tongs.

When compared with a standard product containing cysteine at the same concentration and whose odour is strong and specific, no odour is perceived with composition D of the invention. The process using composition D also gives the hair manageability, a reduction of frizziness and volume, and ease of styling.

As a variant, composition D may be applied after optional shampooing, and left to stand on the hairs for a shorter time, especially 5 minutes, and this operation may be repeated several times successively. Composition D may be applied, for example, between 3 and 5 times consecutively to obtain a reduction in volume and frizziness, manageability of the hair and gentle relaxation, all in a semi-durable manner. This process does not give off any odour.

### Example of a hair-removing composition

Composition F according to the invention was prepared from the ingredients indicated, in grams of active material, in the table below:

| **INCl name** | **F** |
|---|---|
| Urea | 8 |
| Diethylenetriaminepentaacetic acid, pentasodium salt | 0.16 |
| Potassium hydroxide | 0.66 |
| Calcium hydroxide | 3.5 |
| Potassium thioglycolate | 4.86 |
| Mineral oil | 3 |
| Cetearyl alcohol | 7 |
| PPG-5-Ceteth-20 | 3 |
| Mixture of cetearyl alcohol, dicetyl phosphate and oxyethylenated cetyl phosphate (10 OE) (75/14/11) *(Crodafos CES-PA from Croda)* | 5 |
| Water | qs 100 |

This hair-removing composition F gives off little or no sulfur odour on application or during the leave-on time.

## Claims

1. Non-colouring composition comprising:
- one or more sulfureous reducing agents, the sulfureous reducing agent(s) being present in proportions ranging from 1.0% to 50.0% by weight relative to the total weight of the composition;
- one or more phosphoric surfactants;
- one or more nonionic surfactants other than the phosphoric surfactants; and
- from 7% to 60% by weight of non-liquid fatty substance(s), that are not liquid at room temperature (25°C) and at atmospheric pressure (760 mmHg, i.e. 1.013 x 10⁵ Pa), relative to the total weight of the composition, the nonionic surfactant(s) being different from said non-liquid fatty substance(s),
wherein the non-liquid fatty substance(s) comprises fatty alcohols;
- one or more colouring agents which content, if present, does not exceed 0.001% by weight, relative to the total weight of the composition.

2. Composition according to Claim 1, **characterized in that** the sulfureous reducing agent(s) are chosen from thiol-based and non-thiol-based reducing agents, preferably from thiol-based reducing agents.

3. Composition according to either of Claims 1 and 2, **characterized in that** the sulfureous reducing agent(s) are chosen from thioglycolic acid, thiolactic acid, cysteine, cysteamine, homocysteine, glutathione, thioglycerol, thiomalic acid, 2-mercaptopropionic acid, 3-mercaptopropionic acid, thiodiglycol, 2-mercaptoethanol, dithiothreitol, thioxanthine, thiosalicylic acid, thiopropionic acid, lipoic acid and N-acetylcysteine, and salts thereof.

4. Composition according to any one of the preceding claims, **characterized in that** the sulfureous reducing agent(s) are present in proportions ranging from 1.0% to 20.0% by weight, preferably from 2.0% to 15.0% by weight relative to the total weight of the composition.

5. Composition according to any one of the preceding claims, **characterized in that** the phosphoric surfactant(s) have the following formula: 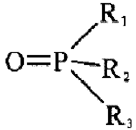 in which:
R₁, R₂ and R₃, which may be identical or different, represent a group chosen from:
- a group -OM, in which M represents a hydrogen atom or an alkali metal, such as Na, Li or K, preferably Na or K;
- a group -OR₄, in which R₄ represents a linear or branched C₁-C₄₀ alkyl group, preferably a C₁₂-C₂₀ alkyl group and more preferably a C₁₆ or C₁₈ alkyl group, a linear or branched C₂-C₄₀ alkenyl group, preferably a C₁₂-C₂₀ alkenyl group and more preferably a C₁₆ or C₁₈ alkenyl group, a C₃-C₄₀ cyclic alkyl group, a C₃-C₄₀ cyclic alkenyl group, a C₅-C₄₀ aromatic group or a C₆-C₄₀ aralkyl group; and
- an oxyalkylene group -(OCH₂CH₂)ₙ(OCH₂CH(CH₃))ₘOR₄ in which R₄ is as defined previously, n represents an integer ranging from 1 to 50 and m represents an integer ranging from 0 to 50,
given that at least one from among R₁, R₂ and R₃ is a group -OM and that at least one from among R₁, R₂ and R₃ is a group -OR₄ or -(OCH₂CH₂)ₙ(OCH₂CH(CH₃))ₘOR₄.

6. Composition according to any one of the preceding claims, in which the phosphoric surfactant(s) are chosen from alkoxylated fatty alcohol phosphates containing from 12 to 20 carbon atoms with from 1 to 50 mol of alkylene oxide chosen from ethylene oxide and propylene oxide, and non-alkoxylated alcohol dialkyl phosphates containing from 12 to 22 carbon atoms, and mixtures thereof, preferably from combinations of ceteth-10 phosphate and dicetyl phosphate, combinations of ceteth-20 phosphate and dicetyl phosphate, and combinations of oleth-5 phosphate and dioleyl phosphate.

7. Composition according to any one of the preceding claims, **characterized in that** the phosphoric surfactant(s) are present in proportions ranging from 0.01% to 20.0% by weight, more preferentially from 0.1% to 15.0% by weight and better still from 0.2% to 10.0% by weight, relative to the total weight of the composition.

8. Composition according to any one of the preceding claims, **characterized in that** the non-liquid fatty substance(s) are chosen from fatty alcohols, fatty acid and/or fatty alcohol esters, non-silicone waxes, fatty amines, fatty ethers and silicones that are non-liquid and preferably solid, and mixtures thereof, and even more preferentially from non-liquid fatty alcohols.

9. Composition according to any one of the preceding claims, **characterized in that** it comprises one or more non-liquid fatty substances in a content ranging from 7.0% to 25% by weight and more preferably from 8% to 25% by weight relative to the total weight of the composition.

10. Composition according to any one of the preceding claims, **characterized in that** it comprises one or more liquid fatty substances, preferably in a content ranging from 0.01% to 20.0% by weight, better still from 0.1% to 15.0% by weight, and even better from 2.0% to 10.0% by weight relative to the total weight of the composition.

11. Composition according to the preceding claim, in which the liquid fatty substance is chosen from hydrocarbons, preferably liquid paraffin or liquid petroleum jelly, and silicones, preferably organomodified silicones, especially polydiarylsiloxanes and polyalkylarylsiloxanes functionalized with organofunctional groups, and preferably phenyl trimethicone.

12. Composition according to any one of the preceding claims, **characterized in that** the weight ratio between the amount of sulfureous reducing agent, on the one hand, and the amount of phosphoric surfactant, on the other hand, is greater than or equal to 0.5 and preferably ranges from 1 to 30 and more preferably from 1 to 20.

13. Composition according to any one of the preceding claims, **characterized in that** the nonionic surfactant(s) other than the phosphoric surfactants are chosen from:
• oxyethylenated and/or oxypropylenated C₈-C₃₀ alcohols comprising from 1 to 100 mol of ethylene oxide and/or of propylene oxide, preferably from 2 to 50 and more particularly from 2 to 30 mol of ethylene oxide and/or of propylene oxide;
• saturated or unsaturated oxyethylenated plant oils comprising from 1 to 100 and preferably from 2 to 50 mol of ethylene oxide;
• (C₈-C₃₀)alkyl(poly)glycosides, which are optionally oxyalkylenated (0 to 10 OE) and comprising 1 to 15 glucose units;
• monoglycerolated or polyglycerolated C₈-C₄₀ alcohols, comprising from 1 to 30 mol of glycerol and preferably from 1 to 10 mol of glycerol;
• saturated or unsaturated, linear or branched, oxyalkylenated C₈-C₃₀ fatty acid amides;
mixtures thereof;
and more preferentially from oxyethylenated and/or oxypropylenated C₈-C₃₀ alcohols comprising from 1 to 100 mol of ethylene oxide and/or of propylene oxide, preferably from 2 to 50 and more particularly from 2 to 30 mol of ethylene oxide and/or of propylene oxide.

14. Composition according to any one of the preceding claims, **characterized in that** the nonionic surfactant(s) other than the phosphoric surfactants are present in proportions ranging from 0.01% to 30.0% by weight, preferably from 0.1% to 20.0% by weight and more preferably from 1.0% to 10.0% by weight, relative to the total weight of the composition.

15. Composition according to any one of the preceding claims, **characterized in that** it comprises at least one cationic polymer, preferably chosen from cationic polysaccharides, especially cationic celluloses and galactomannan gums, cationic cyclopolymers, in particular dimethyldiallylammonium salt homopolymers or copolymers, quaternary polymers of vinylpyrrolidone and of vinylimidazole, optionally crosslinked homopolymers or copolymers of methacryloyloxy(C₁-C₄)alkyltri(C₁-C₄)alkylammonium salts, and mixtures thereof.

16. Process for permanently reshaping, especially for straightening or perming, keratin fibres, in particular human keratin fibres such as the hair, which consists in performing the following steps:
(i) a composition as defined according to any one of Claims 1 to 15 is applied to the said fibres and is left to stand on the fibres for a time sufficient for shaping, and
(ii) an oxidizing composition is optionally applied to the said fibres for a time sufficient for fixing the shape.

17. Use of the composition according to one of Claims 1 to 15, for permanently reshaping the hair.

18. Use of the composition according to one of Claims 1 to 15, for hair removal.

## Patentansprüche

1. Nichtfärbende Zusammensetzung, umfassend:
- ein oder mehrere schwefelhaltige Reduktionsmittel, wobei das schwefelhaltige Reduktionsmittel bzw. die schwefelhaltigen Reduktionsmittel in Anteilen im Bereich von 1,0 bis 50,0 Gew.-%, bezogen auf das Gesamtgewicht der Zusammensetzung, vorliegt bzw. vorliegen;
- ein oder mehrere Phosphortenside;
- ein oder mehrere nichtionische Tenside, die von den Phosphortensiden verschieden sind; und
- 7 bis 60 Gew.-% einer oder mehrerer nichtflüssiger Fettsubstanzen, die bei Raumtemperatur (25 °C) und bei Normaldruck (760 mmHg, d. h. 1,013 × 10⁵ Pa) nicht flüssig sind, bezogen auf das Gesamtgewicht der Zusammensetzung,
wobei das nichtionische Tensid bzw. die nichtionischen Tenside von der nichtflüssigen Fettsubstanz bzw. den nichtflüssigen Fettsubstanzen verschieden ist bzw. sind,
wobei die nichtflüssige Fettsubstanz bzw. die nichtflüssigen Fettsubstanzen Fettalkohole umfasst bzw. umfassen;
- ein oder mehrere Farbmittel, deren Gehalt, sofern vorhanden, nicht mehr als 0,001 Gew.-%, bezogen auf das Gesamtgewicht der Zusammensetzung, beträgt.

2. Zusammensetzung nach Anspruch 1, **dadurch gekennzeichnet, dass** das schwefelhaltige Reduktionsmittel bzw. die schwefelhaltigen Reduktionsmittel aus auf Thiol basierenden und nicht auf Thiol basierenden Reduktionsmitteln, vorzugsweise aus auf Thiol basierenden Reduktionsmitteln, ausgewählt ist bzw. sind.

3. Zusammensetzung nach einem der Ansprüche 1 und 2, **dadurch gekennzeichnet, dass** das schwefelhaltige Reduktionsmittel bzw. die schwefelhaltigen Reduktionsmittel aus Thioglykolsäure, Thiomilchsäure, Cystein, Cysteamin, Homocystein, Glutathion, Thioglycerin, Thioäpfelsäure, 2-Mercaptopropionsäure, 3-Mercaptopropionsäure, Thiodiglykol, 2-Mercaptoethanol, Dithiothreitol, Thioxanthin, Thiosalicylsäure, Thiopropionsäure, Liponsäure und N-Acetylcystein und Salzen davon ausgewählt ist bzw. sind.

4. Zusammensetzung nach einem der vorhergehenden Ansprüche, **dadurch gekennzeichnet, dass** das schwefelhaltige Reduktionsmittel bzw. die schwefelhaltigen Reduktionsmittel in Anteilen im Bereich von 1,0 bis 20,0 Gew.-%, vorzugsweise von 2,0 bis 15,0 Gew.-%, bezogen auf das Gesamtgewicht der Zusammensetzung, vorliegt bzw. vorliegen.

5. Zusammensetzung nach einem der vorhergehenden Ansprüche, **dadurch gekennzeichnet, dass** das Phosphortensid bzw. die Phosphortenside die folgende Formel aufweist bzw. aufweisen: 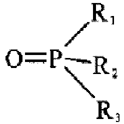 in der:
R₁, R₂ und R₃, die gleich oder verschieden sein können, für eine Gruppe stehen, die aus
- einer Gruppe -OM, in der M für ein Wasserstoffatom oder ein Alkalimetall, wie Na, Li oder K, vorzugsweise Na oder K, steht;
- einer Gruppe -OR₄, in der R₄ für eine lineare oder verzweigte C₁-C₄₀-Alkylgruppe, vorzugsweise eine C₁₂-C₂₀-Alkylgruppe und weiter bevorzugt eine C₁₆- oder C₁₈-Alkylgruppe, eine lineare oder verzweigte C₂-C₄₀-Alkenylgruppe, vorzugsweise eine C₁₂-C₂₀-Alkenylgruppe und weiter bevorzugt eine C₁₆- oder C₁₈-Alkenylgruppe, eine C₃-C₄₀-Cycloalkylgruppe, eine C₃-C₄₀-Cycloalkenylgruppe, eine aromatische C₅-C₄₀-Gruppe oder eine C₆-C₁₄-Aralkylgruppe steht; und
- einer Oxyalkylengruppe - (OCH₂CH₂)ₙ(OCH₂CH(CH₃))ₘOR₄, in der R₄ wie oben definiert ist, n für eine ganze Zahl im Bereich von 1 bis 50 steht und m für eine ganze Zahl im Bereich von 0 bis 50 steht;
ausgewählt ist, mit der Maßgabe, dass mindestens eines von R₁, R₂ und R₃ für eine Gruppe -OM steht und dass mindestens eines von R₁, R₂ und R₃ für eine Gruppe -OR₄ oder -(OCH₂CH₂)ₙ(OCH₂CH(CH₃))ₘOR₄ steht.

6. Zusammensetzung nach einem der vorhergehenden Ansprüche, wobei das Phosphortensid bzw. die Phosphortenside aus alkoxylierten Fettalkoholphosphaten, mit 12 bis 20 Kohlenstoffatomen und 1 bis 50 mol Alkylenoxid, das aus Ethylenoxid und Propylenoxid ausgewählt ist, und nicht alkoxylierten Alkoholdialkylphosphaten mit 12 bis 22 Kohlenstoffatomen und Mischungen davon, vorzugsweise aus Kombinationen von Ceteth-10-phosphat und Dicetylphosphat, Kombinationen von Ceteth-20-phosphat und Dicetylphosphat und Kombinationen von Oleth-5-phosphat und Dioleylphosphat, ausgewählt ist bzw. sind.

7. Zusammensetzung nach einem der vorhergehenden Ansprüche, **dadurch gekennzeichnet, dass** das Phosphortensid bzw. die Phosphortenside in Anteilen im Bereich von 0,01 bis 20,0 Gew.-%, weiter bevorzugt von 0,1 bis 15,0 Gew.-% und noch besser von 0,2 bis 10 Gew.-%, bezogen auf das Gesamtgewicht der Zusammensetzung, vorliegt bzw. vorliegen.

8. Zusammensetzung nach einem der vorhergehenden Ansprüche, **dadurch gekennzeichnet, dass** die nichtflüssige Fettsubstanz bzw. die nichtflüssigen Fettsubstanzen aus Fettalkoholen, Fettsäure- und/oder Fettalkoholestern, Nichtsilikonwachsen, Fettaminen, Fettethern und Silikonen, die nichtflüssig und vorzugsweise fest sind, und Mischungen davon und noch weiter bevorzugt aus nichtflüssigen Fettalkoholen ausgewählt ist bzw. sind.

9. Zusammensetzung nach einem der vorhergehenden Ansprüche, **dadurch gekennzeichnet, dass** sie eine oder mehrere nichtflüssige Fettsubstanzen in einem Gehalt im Bereich von 7,0 bis 25 Gew.-% und weiter bevorzugt von 8 bis 25 Gew.-%, bezogen auf das Gesamtgewicht der Zusammensetzung, umfasst.

10. Zusammensetzung nach einem der vorhergehenden Ansprüche, **dadurch gekennzeichnet, dass** sie eine oder mehrere flüssige Fettsubstanzen umfasst, vorzugsweise in einem Gehalt im Bereich von 0,01 bis 20 Gew.-%, noch besser von 0,1 bis 15 Gew.-% und noch besser von 2,0 bis 10,0 Gew.-%, bezogen auf das Gesamtgewicht der Zusammensetzung.

11. Zusammensetzung nach dem vorhergehenden Anspruch, wobei die flüssige Fettsubstanz aus Kohlenwasserstoffen, vorzugsweise Flüssigparaffin oder Vaselineöl, und Silikonen, vorzugsweise organisch modifizierten Silikonen, insbesondere Polydiarylsiloxanen und Polyalkylarylsiloxanen, die mit organofunktionellen Gruppen funktionalisiert sind, und vorzugsweise Phenyltrimethicon, ausgewählt ist.

12. Zusammensetzung nach einem der vorhergehenden Ansprüche, **dadurch gekennzeichnet, dass** das Gewichtsverhältnis zwischen der Menge an schwefelhaltigem Reduktionsmittel einerseits und der Menge an Phosphortensid andererseits größer oder gleich 0,5 ist und vorzugsweise im Bereich von 1 bis 30 weiter bevorzugt von 1 bis 20 liegt.

13. Zusammensetzung nach einem der vorhergehenden Ansprüche, **dadurch gekennzeichnet, dass** das nichtionische Tensid bzw. die nichtionischen Tenside, das bzw. die von den Phosphortensiden verschieden ist bzw. sind, aus
• oxyethylenierten und/oder oxypropylenierten C₈-C₃₀-Alkoholen mit 1 bis 100 mol Ethylenoxid und/oder Propylenoxid, vorzugsweise 2 bis 50 und weiter bevorzugt 2 bis 30 mol Ethylenoxid und/oder Propylenoxid;
• gesättigten oder ungesättigten oxyethylenierten Pflanzenölen mit 1 bis 100 und vorzugsweise 2 bis 50 mol Ethylenoxid;
• (C₈-C₃₀)Alykl (poly) glykosiden, die gegebenenfalls oxyalkyleniert sind (0 bis 10 OE) und 1 bis 15 Glucoseeinheiten umfassen;
• monoglycerinierten und polyglycerinierten C₈-C₄₀-Alkoholen mit 1 bis 30 mol Glycerin und vorzugsweise 1 bis 10 mol Glycerin;
• gesättigten oder ungesättigten, linearen oder verzweigten oxyalkylenierten C₈-C₃₀-Fettsäure-amiden;
Mischungen davon
und weiter bevorzugt aus oxyethylenierten und/oder oxypropylenierten C₈-C₃₀-Alkoholen mit 1 bis 100 mol Ethylenoxid und/oder Propylenoxid, vorzugsweise 2 bis 50 und weiter bevorzugt 2 bis 30 mol Ethylenoxid und/oder Propylenoxid
ausgewählt ist bzw. sind.

14. Zusammensetzung nach einem der vorhergehenden Ansprüche, **dadurch gekennzeichnet, dass** das nichtionische Tensid bzw. die nichtionischen Tenside, das bzw. die von den Phosphortensiden verschieden ist bzw. sind, in Anteilen im Bereich von 0,01 bis 30 Gew.-%, vorzugsweise von 0,1 bis 20,0 Gew.-% und weiter bevorzugt von 1,0 bis 10,0 Gew.-%, bezogen auf das Gesamtgewicht der Zusammensetzung, vorliegt bzw. vorliegen.

15. Zusammensetzung nach einem der vorhergehenden Ansprüche, **dadurch gekennzeichnet, dass** sie mindestens ein kationisches Polymer umfasst, das vorzugsweise aus kationischen Polysacchariden, insbesondere kationischen Cellulosen und Galactomannangummen, kationischen Cyclopolymeren, insbesondere Dimethyldiallylammoniumsalz-Homopolymeren oder -Copolymeren, quaternären Polymeren von Vinylpyrrolidon und Vinylimidazol, gegebenenfalls vernetzten Copolymeren von Methacryloyloxy(C₁-C₄)-alkyltri(C₁-C₄)alkylammoniumsalzen und Mischungen davon, ausgewählt ist.

16. Verfahren zur dauerhaften Verformung, insbesondere zum Glätten oder Legen einer Dauerwelle, von Keratinfasern, insbesondere menschlichen Keratinfasern wie dem Haar, das darin besteht, dass man die folgenden Schritte durchführt:
(i) eine Zusammensetzung gemäß einem der Ansprüche 1 bis 15 wird auf die Fasern aufgebracht und über einen für die Formung ausreichenden Zeitraum einwirken gelassen, und
(ii) gegebenenfalls wird eine oxidierende Zusammensetzung über einen zum Fixieren der Form ausreichenden Zeitraum auf die Fasern aufgebracht.

17. Verwendung der Zusammensetzung nach einem der Ansprüche 1 bis 15 zur dauerhaften Verformung des Haares.

18. Verwendung der Zusammensetzung nach einem der Ansprüche 1 bis 15 zur Haarentfernung.

## Revendications

1. Composition non colorante comprenant :
- un ou plusieurs agents de réduction soufrés, l'agent ou les agents de réduction soufrés étant présents en des proportions dans la plage de 1,0 % à 50,0 % en poids par rapport au poids total de la composition ;
- un ou plusieurs tensioactifs phosphoriques ;
- un ou plusieurs tensioactifs non ioniques différents des tensioactifs phosphoriques ; et
- de 7 % à 60 % en poids de corps gras, qui sont non liquides à température ambiante (25 °C) et à pression atmosphérique (760 mmHg, c'est-à-dire 1,013 x 10⁵ Pa), par rapport au poids total de la composition,
le(s) tensioactif(s) non ionique(s) étant différent(s) de ladite/desdits corps gras non liquide(s),
le/les corps gras non liquide(s) comprenant des alcools gras ;
- un ou plusieurs agents colorants dont la teneur, s'il est/s'ils sont présent(s), n'excède pas 0,001 % en poids, par rapport au poids total de la composition.

2. Composition selon la revendication 1, **caractérisée en ce que** l'agent ou les agents de réduction soufré (s) sont choisis parmi des agents de réduction à base de thiol et non à base de thiol, préférablement parmi des agents de réduction à base de thiol.

3. Composition selon l'une ou l'autre des revendications 1 et 2, **caractérisée en ce que** l'agent ou les agents de réduction soufré(s) sont choisis parmi l'acide thioglycolique, l'acide thiolactique, la cystéine, la cystéamine, l'homocystéine, le glutathion, le thioglycérol, l'acide thiomalique, l'acide 2-mercaptopropionique, l'acide 3-mercaptopropionique, le thiodiglycol, le 2-mercaptoéthanol, le dithiothréitol, la thioxanthine, l'acide thiosalicylique, l'acide thiopropionique, l'acide lipoïque et la N-acétylcystéine, et des sels correspondants.

4. Composition selon l'une quelconque des revendications précédentes, **caractérisée en ce que** l'agent ou les agents de réduction soufré(s) sont présents en des proportions dans la plage de 1,0 % à 20,0 % en poids, préférablement de 2,0 % à 15,0 % en poids par rapport au poids total de la composition.

5. Composition selon l'une quelconque des revendications précédentes, **caractérisée en ce que** le(s) tensioactif(s) phosphorique(s) possède(nt) la formule suivante : 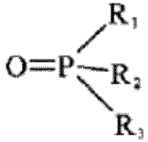 dans laquelle :
R₁, R₂ et R₃, qui peuvent être identiques ou différents, représentent un groupe choisi parmi :
- un groupe -OM, dans lequel M représente un atome d'hydrogène ou un métal alcalin, tel que Na, Li ou K, préférablement Na ou K ;
- un groupe -OR₄, dans lequel R₄ représente un groupe C₁₋₄₀-alkyle linéaire ou ramifié, préférablement un groupe C₁₂₋₂₀-alkyle et plus préférablement un groupe C₁₆-alkyle ou C₁₈-alkyle, un groupe C₂₋₄₀-alcényle linéaire ou ramifié, préférablement un groupe C₁₂₋₂₀-alcényle et plus préférablement un groupe C₁₆-alcényle ou C₁₈-alcényle, un groupe C₃₋₄₀-alkyle cyclique, un groupe C₃₋₄₀-alcényle cyclique, un groupe C₅₋₄₀-aromatique ou un groupe C₆₋₄₀-aralkyle ; et
- un groupe oxyalkylène - (OCH₂CH₂)ₙ(OCH₂CH(CH₃))ₘOR₄ dans lequel R₄ est tel que défini précédemment, n représente un entier dans la plage de 1 à 50 et m représentent un entier dans la plage de 0 à 50,
sachant qu'au moins l'un parmi R₁, R₂ et R₃ est un groupe -OM et qu'au moins l'un parmi R₁, R₂ et R₃ est un groupe -OR₄ ou - (OCH₂CH₂)ₙ(OCH₂CH(CH₃))ₘOR₄.

6. Composition selon l'une quelconque des revendications précédentes, dans laquelle le(s) tensioactif(s) phosphorique(s) sont choisis parmi des phosphates d'alcool gras contenant de 12 à 20 atomes de carbone alcoxylé avec de 1 à 50 moles d'oxyde d'alkylène choisi parmi l'oxyde d'éthylène et l'oxyde de propylène, et des dialkylphosphates d'alcool non alcoxylé contenant de 12 à 22 atomes de carbone, et des mélanges correspondants, préférablement parmi des combinaisons de cététh-10 phosphate et de dicétylphosphate, des combinaisons de cététh-20 phosphate et de dicétylphosphate, et des combinaisons d'oléth-5 phosphate et de dioléylphosphate.

7. Composition selon l'une quelconque des revendications précédentes, **caractérisée en ce que** le(s) tensioactif(s) phosphorique(s) sont présents en des proportions dans la plage de 0,01 % à 20,0 % en poids, plus préférentiellement de 0,1 % à 15,0 % en poids et encore mieux de 0,2 % à 10,0 % en poids, par rapport au poids total de la composition.

8. Composition selon l'une quelconque des revendications précédentes, **caractérisée en ce que** le ou les corps non liquide(s) sont choisies parmi des alcools gras, des esters d'acide gras et/ou d'alcool gras, des cires non de silicone, des amines grasses, des éthers gras et des silicones qui sont non liquides et préférablement solides, et des mélanges correspondants, et encore plus préférentiellement parmi des alcools gras non liquides.

9. Composition selon l'une quelconque des revendications précédentes, **caractérisée en ce qu'**elle comprend un ou plusieurs corps gras non liquides en une teneur dans la plage de 7,0 % à 25 % en poids et plus préférablement de 8 % à 25 % en poids par rapport au poids total de la composition.

10. Composition selon l'une quelconque des revendications précédentes, **caractérisée en ce qu'**elle comprend un ou plusieurs corps gras liquides, préférablement en une teneur dans la plage de 0,01 % à 20,0 % en poids, encore mieux de 0,1 % à 15,0 % poids, et mieux encore de 2,0 % à 10,0 % en poids par rapport au poids total de la composition.

11. Composition selon la revendication précédente, dans laquelle le corps gras liquide est choisi parmi des hydrocarbures, préférablement de la paraffine liquide ou de la gelée de pétrole liquide, et des silicones, préférablement des silicones organomodifiées, notamment des polydiarylsiloxanes et des polyalkylarylsiloxanes fonctionnalisés par des groupes organofonctionnels, et préférablement le phényltriméthicone.

12. Composition selon l'une quelconque des revendications précédentes, **caractérisée en ce que** le rapport pondéral entre la quantité d'agent de réduction soufrés, d'un côté, et la quantité de tensioactif phosphorique, de l'autre, est supérieur ou égal à 0,5 et préférablement est dans la plage de 1 à 30 et plus préférablement de 1 à 20.

13. Composition selon l'une quelconque des revendications précédentes, **caractérisée en ce que** le(s) tensioactif(s) non ionique(s) différent(s) des tensioactifs phosphoriques sont choisis parmi :
• des C₈₋₃₀-alcools oxyéthylénés et/ou oxypropylénés comprenant de 1 à 100 moles d'oxyde d'éthylène et/ou d'oxyde de propylène, préférablement de 2 à 50 et plus particulièrement de 2 à 30 moles d'oxyde d'éthylène et/ou d'oxyde de propylène ;
• des huiles végétales oxyéthylénées saturées ou insaturées comprenant de 1 à 100 et préférablement de 2 à 50 moles d'oxyde d'éthylène ;
• des C₈₋₃₀-alkyl(poly)glycosides, qui sont éventuellement oxyalkylénés (0 à 10 OE) et comprenant 1 à 15 motifs glucose ;
• des C₈₋₄₀-alcools monoglycérolés ou polyglycérolés, comprenant de 1 à 30 moles de glycérol et préférablement de 1 à 10 moles de glycérol ;
• des amides d'acides gras en C₈₋₃₀ oxyalkylénés, saturés ou insaturés, linéaires ou ramifiés ;
des mélanges correspondants ;
et plus préférentiellement parmi des C₈₋₃₀-alcools oxyéthylénés et/ou oxypropylénés comprenant de 1 à 100 moles d'oxyde d'éthylène et/ou d'oxyde de propylène, préférablement de 2 à 50 et plus particulièrement de 2 à 30 moles d'oxyde d'éthylène et/ou d'oxyde de propylène.

14. Composition selon l'une quelconque des revendications précédentes, **caractérisée en ce que** le(s) tensioactif(s) non ionique(s) différent(s) des tensioactifs phosphoriques sont présents en des proportions dans la plage de 0,01 % à 30,0 % en poids, préférablement de 0,1 % à 20,0 % en poids et plus préférablement de 1,0 % à 10,0 % en poids, par rapport au poids total de la composition.

15. Composition selon l'une quelconque des revendications précédentes, **caractérisée en ce qu'**elle comprend au moins un polymère cationique, préférablement choisi parmi des polysaccharides cationiques, notamment des celluloses cationiques et des gommes de galactomannane, des cyclopolymères cationiques, en particulier des homopolymères ou des copolymères de sel de diméthyldiallylammonium, des polymères quaternaires de vinylpyrrolidone et de vinylimidazole, éventuellement des homopolymères ou des copolymères réticulés de sels de méthacryloyloxy-C₁₋₄-alkyltri-C₁₋₄-alkylammonium, et des mélanges correspondants.

16. Procédé pour la déformation permanente, notamment pour le défrisage ou l'ondulation permanente, de fibres de kératine, en particulier de fibres de kératine humaines telles que les cheveux, qui est constitué par la réalisation des étapes suivantes :
(i) une composition telle que définie selon l'une quelconque des revendications 1 à 15 est appliquée auxdites fibres et laissée à poser sur les fibres pendant un temps suffisant pour la mise en forme, et
(ii) une composition oxydante est éventuellement appliquée auxdites fibres pendant un temps suffisant pour la fixation de la forme.

17. Utilisation de la composition selon l'une des revendications 1 à 15, pour la déformation permanente des cheveux.

18. Utilisation de la composition selon l'une des revendications 1 à 15, pour l'épilation.
